# EUROPEAN PATENT SPECIFICATION

(11) **EP 1 401 551 B1**
(45) Date of publication and mention of the grant of the patent: **21.06.2006**
(21) Application number: 02748989.7
(22) Date of filing: 01.07.2002
(51) Int. Cl.: B01D 9/00

(54) **NANOPARTICLE STRUCTURES**
NANOPARTIKEL-STRUKTUREN
STRUCTURES DE NANOPARTICULE

(30) Priority: 29.06.2001 GB 0116074
(43) Date of publication of application: 31.03.2004
(73) Proprietor: University of Strathclyde, Glasgow G1 1YQ, Scotland (GB)
(72) Inventor: MOORE, Barry Douglas, Crosshill, Glasgow G42 8DT (GB); CUNNINGHAM, Douglas Burns, Glasgow G44 3AT (GB)
(74) Representative: Chapman, Paul Gilmour
(86) International application number: PCT/GB2002/003024
(87) International publication number: WO 2003/002225

(56) References cited:
- EP-A- 0 972 563
- WO-A-00/69887
- WO-A-00/77281
- DE-A- 19 619 287

## Description

### Backqround of the Invention

This invention relates to nanoparticle structures such as assemblies of metals and/or semi-conductor nanoparticles on the surfaces of crystalline substrates.

Nanoscale particles, so-called nanoparticles, hold promise for use as advanced materials with new electronic, magnetic, optic and thermal properties, as well as new catalytic properties. One reason for this is the quantum size effect, which is derived from the dramatic reduction of the number of free electrons in particles in the range 1 - 10 nm. In addition their properties can often be tuned via the functional groups displayed at the particle surface. However, accurate control of the particle size is important to provide novel physical and/or chemical properties. Functional groups may be introduced via ligands that are often bound to the nanoparticle material in order to stabilise the nanoparticles.

Moreover, ordered assemblies of nanoparticles with defined 2-dimensional and/or 3-dimensional spatial configurations are anticipated to display novel properties that are not present in isolated particles.

Previous methods of producing 2-dimensional and 3-dimensional structures have involved the of nanoparticles by deposition of a few drops of their dispersion on a substrate, or by dipping the substrate into the dispersion and the formation of 2-dimensional Au and Ag monolayers have been reported. Another approach is to use external forces to obtain nanoparticle monolayers, such as an electrophorectic deposition, microfluidics, Langmuir Blodgett technique, and DNA hybridisation. Nevertheless control of the size and build-up of nanoparticles assemblies is important in certain applications and this has proved difficult. One method of controlling the size of nanoparticle aggregates has been to form them inside other matrices such as polymers. However, while this may serve to control the size of the nanopaticles assemblies, it is not clear how such nanoparticles may be ordered, for example into fine lines of nanometre dimensions.

Previous methods for making a wide-range of different types of nanoparticles and altering their surface properties so that they form good dispersions in different solvents are known in the art. Surface properties can be controlled by ligands which bind to the nanoparticle material such as a metal and display a specific group with desirable physical or chemical properties at the outer surface of the particle. For example, using long-chain alkyl thiols as the surface coating for gold nanoparticles, the nanoparticles may be made soluble in non-polar solvents such as toluene and poorly insoluble in water, while with polar thiols like tiopronin, gold nanoparticles soluble in water but insoluble in polar solvents can be made.

WO-A-0069887 relates to protein-coated microcrystals and their method of preparation. In particular, WO-A-0069887 relates to water soluble particles comprising a biological macromolecule and a method of isolating a biological macromolecule from an aqueous solution with simultaneous dehydration of the protein, to provide protein biological macromolecule particles.

WO-A-0077281 relates to a process for preparing coated crystals by coating crystal template particles with alternated layers of oppositely charged polyelectrolytes and/or nanoparticles. The process involves a two-stage process of providing a dispersion of crystal template particles in a solvent and then coating said particles with a multilayer comprising alternating layers of oppositely charged polyelectrolyte and/or nanoparticles.

It is clear therefore that there is the need in the art to provide novel nanoparticle structures such as ordered arrays of, for example, metal or semi-conductor nanoparticles and simple and efficient methods of preparing them.

It is an object of at least one aspect of the present invention to obviate and/or mitigate at least one of the aforementioned problems.

### Summary of the Invention

Generally speaking, the present invention is based on the observation by the inventors that coprecipitation of a solution or dispersion of nanoparticles together with a crystal forming material yields crystals, the surfaces of which are coated with an array (i.e. assembly of nanoparticles.

According to a first aspect there is provided a method of preparing nanoparticle coated crystals comprising the steps of:
(a) providing a mixture comprising nanoparticles and a solution of a crystal forming material; and
(b) coprecipitating the nanoparticles and the crystal forming material such that crystals are formed, a surface or surfaces of which are at least partially coated with nanoparticles; and
wherein the nanoparticles are provided as a dispersion or solution and are comprised of one of or a combination of any of the following: metals, metal alloys, semi-metals, semi-conductors, carbon allotropes, insulators and mixtures thereof.

Without wishing to be bound by theory the nanoparticles are thought to be attached to the crystal surface bound to the crystals via non-covalent interactions using, for example, van-der Waals and electrostatic interactions. Coprecipitating means that both the crystal forming material and nanoparticles precipitate out of solution together.

Any rapid coprecipitation method such as use of a non-solvent, solvent evaporation, temperature reduction etc., is suitable. However, a generally rapid coprecipitation is essential for the process. For example, a large excess of non-solvent may be rapidly mixed with the aqueous solution of nanoparticles and crystal forming materials. Alternatively, the solution of nanoparticles and crystal forming material may be added dropwise or via a spray technique to a non-solvent.

It is to be understood that the term "coprecipitation" refers to the process in which both the nanoparticles and crystal forming material precipitate out of solution together, forming crystals, a surface or surfaces of which are at least partially coated with nanoparticles. The term crystal is intended to mean a three-dimensional shape comprising planar surfaces and is thus distinguished from generally spherical or spheroid shaped amorphous particles.

The nanoparticles in solution may have a protective monolayer coating (e.g. of a ligand) on their outer surface. The protective coating prevents the nanoparticles from coalescing. The nanoparticles generally have a maximum cross-section of about 0.5 nm - 250 nm, 1 - 20 nm or about 2 - 10 nm with a size distribution of about a mean value ± 20% or preferably of about ± 50%. By varying the chemical nature of the coating the nanoparticles can be dissolved in a variety of different solvents or tuned to specific requirements. The ligand may be any suitable O, S or N - containing organic molecule, particularly suitable are long alkyl chain alcohols, thiols or amines such as nitrogen containing ligands: alkyl amines, cetyltrimethylammonium bromide and other cationic surfactants oxgen containing: polyethylene oxide polymers, cyclodextrins, anionic surfactants also alkylphosphines. Preferably, the coating is a thiol such a N-(mercapto-propionyl)glycine (ie. tiopronin).

Typically, the solubility of the crystal forming material and the nanoparticles are 'matched', i.e., they are both fairly soluble in one solvent and both nearly insoluble in the non-solvent. The solvent in which the crystal forming material and nanoparticles are dissolved together should be fully or partially miscible with the non-solvent used in the coprecipitation.

The nanoparticles in solution may typically form a state in between that of a colloid and a true solution (Langmuir, 1999, 15, 66-76).

Depending upon the ratio of nanoparticles to crystal forming material the coating may either take the form of a close packed assembly of nanoparticles or alternatively more open structures such as in the form of a patterned array. It is understood that the term "patterned array" is taken to mean that the nanoparticles assemble into 2-dimensional or 3-dimensional structures such as lines of regular width which may be straight, curved, branched and/or possess orthogonal or angled junctions and, lines that lie parallel to other lines, repeating patterns, lattices, ridges, blocks and the like. The percentage of surface coverage may vary from 10 - 100% and may be in the form of a monolayer, a bilayer or a multilayer. Preferably, the surface coverage is greater than 20%, 50% or 80%.

If the nanoparticles are deposited as lines, the lines of nanoparticles may have a width of 0.5 to 100 nm and/or a height of 0.5 to 100 nm and/or a length of 1 to 5000 nm. The lines may be made of continuous or discontinuous chains of nanoparticles such that the width and height depends on the diameter of the nanoparticles.

During a typical coprecipitation process >10¹² crystals coated with nanoparticles may be formed. This makes the process much more efficient that other methods which coat only a very limited number of surfaces at a time. Another advantage is the much higher surface area covered. Furthermore, the percentage of surface coverage may be altered by varying the ratio of nanoparticles to crystals.

Conveniently the crystals may be entirely coated with nanoparticles, although this need not be the case and in certain embodiments only portions of the crystals may be coated.

Without wishing to be bound by theory the coating mechanism is believed to occur via the nanoparticles being initially trapped inside a precipitated aggregate mass of crystal forming material. On formation of crystals from the unstable aggregate, the nanoparticles are thus forced to the surface of the crystal lattice structures. The coating of nanoparticles so formed then inhibits any further growth of the crystal structures.

Furthermore, the nanoparticles may be organised to form a patterned array either themselves or in combination with functional molecules.

The nanoparticles may be provided for example as a dispersion or solution and be comprised of metals, metal alloys, semi-metals, semi-conductors, carbon allotropes, insulators or mixtures thereof. Alternatively, the nanoparticles may be provided as a powder which is mixed with the solution of crystal forming material. Examples of suitable metals, alloys or semi-metals include gold, silver, platinum, palladium, and cobalt; and alloys thereof.

Suitable semi-conductors include CdS and ZnS.

The carbon may be in the form of fullerenes (eg. C₆₀) or carbon nanotubes for example.

Insulators may take the form of organic or inorganic dendrimers or hyperbranched polymers such as Starburst (Registered Trade Mark) (PAMAM) Dendrimer Generation 4 (amine terminated) and Starburst (Registered Trade Mark) PAMAM Dendrimer Generation 4.5 (carboxyl terminated).

Any suitable crystal forming material may be used providing that crystals may be formed by precipitation on mixing with or addition to a non-solvent or weak solvent. Preferable crystal-forming materials will tend to be those with high lattice energies and fast crystallisation kinetics. High lattice energies and fast crystallisation kinetics force the nanoparticles to the crystals surface (as described above).

In this manner, the crystals may, for example, be water-soluble ionic materials such as inorganic salts (e.g. KCl, K₂SO₄) highly polar or ionic compounds such as zwitterions (e.g. amino-acids), organic salts, (e.g. sodium glutamate) and sugars (e.g. lactose). Furthermore inorganic or organic salts soluble in polar or intermediate organic solvents but not in non-polar solvents are also suitable (e.g. LiClO₄, NaBF₄. Alternatively, the crystals may be materials soluble in organic solvents, such as aromatic compounds (e.g. naphthalene). The crystal forming material may be provided as a substantially saturated or near saturated or highly concentrated solution.

Typically, the crystals are of nanometre-micrometre dimensions. That is, each face of the crystal may be of the order of 50 nm - 100 µm, such as 50 nm - 10 µm across. The crystals may be of any shape for example cubic, rhombic and the like.

In some cases, for example, gold nanoparticles coated with short thiol chains and coated on K₂SO₄ crystals it is possible to fuse adjacent nanoparticles together on the crystal surface by heating them up to fairly moderate temperatures such as greater than about 230°C. This can be done because, in general, nanoparticles have quite high surface energies stabilised by the surface coatings ie. they want to fuse together but are held apart by the coating. If sufficient heat can be supplied to overcome the activation energy without melting the underlying crystal the nanoparticles will spontaneously fuse with their neighbouring particles on the surface. This can be followed by differential scanning calorimetry (DSC). After fusing together the nanoparticles coated on the crystals, the crystal may be dissolved by placing in an appropriate solvent, so as to leave behind hollow crystal-shaped structures of nanoparticles, such as wire or tube-like structures, sheets, lattices or boxes.

The non-solvent/weak solvent may be selected from, for example, organic liquids comprising of polar solvents (e.g. ethanol, propanol, acetone, acetonitrile, dimethylformamide) intermediate solvents (e.g. ethyl acetate, tetrahydrofuran) or nonpolar solvents (e.g. toluene, hexane) and mixtures thereof, near-critical and super-critical fluids (e.g. carbon dioxide) and/or acids and bases, such as aqueous acids (e.g. HCl (aq)), aqueous bases (e.g. NaOH), organic acids (e.g. acetic acid) and organic bases (e.g. pyridine). The nanoparticles and the crystal forming material should be essentially insoluble in the non-solvent used during the process so that both components coprecipitate when the solution of nanoparticles and crystal forming material is rapidly mixed with the non-solvent. If the crystal forming material is initially partially soluble in the non-solvent it is preferable to pre-saturate the non-solvent with the crystal forming material before carrying out the process. Similarly it is possible to pre-saturate the solvent with the nanoparticles.

Improved results may be obtained during coprecipitation from water if the non-solvent initially contains low levels of water (0.5 - 2%) and is then saturated with the coprecipitant. This is because the solubility of the coprecipitant is often significantly increased relative to dry solvent and it is preferable to ensure the solvent is near saturation in coprecipitant throughout the whole of the precipitation process. This ensures precipitation is rapid and near complete.

The ordered array of nanoparticles coated on the surface of the crystals is readily accessible for modification by chemical, biochemical or photochemical reactions. For example, some or all of the surface groups on the nanoparticles can be exchanged or reacted with compounds in solution or the gas phase. This may alter the physical properties of the particles (ie. both the nanoparticles and the coated crystal particle) such as for example, the overall charge.

Ordered arrays of nanoparticles can also act as templates for organisation of secondary layers of nanoparticles or molecules adsorbed from solution or the gas phase. For example, semiconductor nanoparticles with the correct complementary surface properties can be induced to adsorb on or in between lines of metal nanoparticles. This can give rise to complex nanostructures with unusual electronic properties.

Further steps may include the coating of different nanoparticles from a different solution and appropriate coprecipitation and/or the fusing of further molecules to the nanoparticles.

### Brief Description of the Drawings

Embodiments of the present invention will now be described, by way of non-binding example only, with reference to the accompanying drawings in which:
Figure 1 is a representation of standard crystallisation process according to the prior art;
Figure 2 is a representation of the actual route of co-precipitation according to the present invention;
Figure 3 is a representation of two expected routes of co-precipitation;
Figure 4 is an image taken with a transmission electron microscope of a first group of gold colloid coated crystals;
Figure 5a is an image taken with a transmission electron microscope of a second group of gold nanoparticle coated crystals;
Figure 5b is an expanded view of part of Figure 5a;
Figure 5c is an even further expanded view of part of Figure 5a;
Figure 6 is an image taken with a transmission electron microscope of a third group of larger gold nanoparticle coated crystals;
Figure 7 is an image taken with a transmission electron microscope of a single gold nanoparticle coated crystal;
Figure 8 is an image taken with a transmission electron microscope of a further group of gold nanoparticle coated crystals after fusion;
Figure 9 is an image taken with a scanning electron microscope of gold nanoparticle coated valine crystals;
Figure 10a is an image taken with a scanning electron microscope of gold nanoparticle coated rubidium sulphate crystals;
Figure 10b is an expanded view of part of Figure 10a;
Figure 10c is a transmission electron microscope image of gold nanoparticle coated rubidium sulphate crystals;
Figure 10d is an expanded view of part of Figure 10c;
Figure 11 is a representation of a mixed nanoparticle array on a microcrystal surface which undergoes cross-linking;
Figure 12 is a representation of a bilayer of cross-linked nanoparticles;
Figure 13a is a representation of conventional combination of two different kinds of nanoparticles;
Figure 13b is a representation of combining two different nanoparticles to form anisotropic particles according to the present invention;
Figure 14a is a representation of a conventional method of attempting to form nanoparticles at a polymer surface; and
Figure 14b is a representation of forming nanoparticles at a polymer surface using nanoparticle coated microcrystals according to the present invention.

### Detailed Description

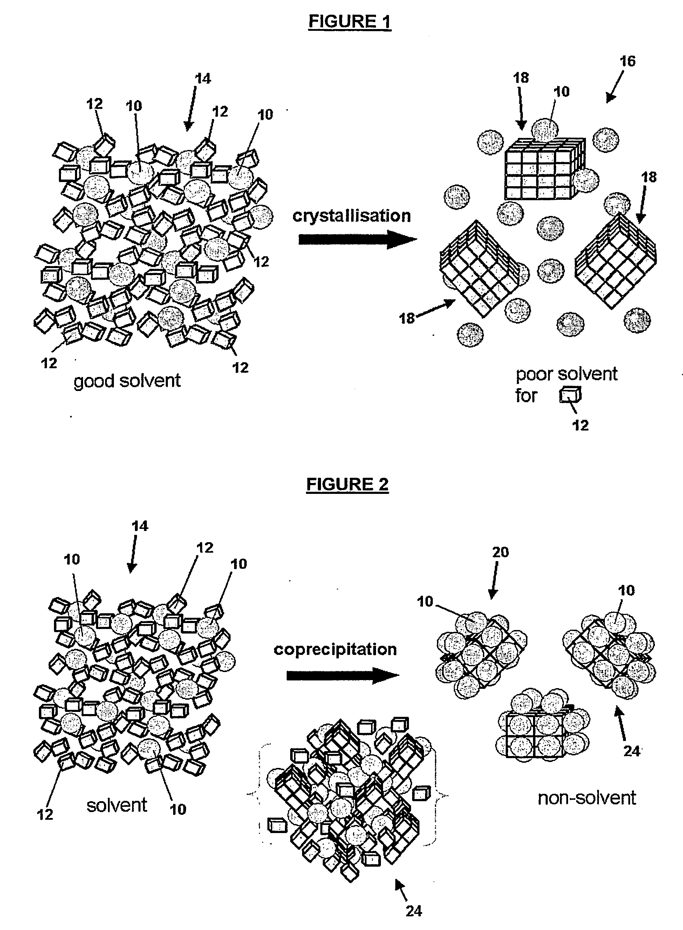

Figure 1 is a representation of a standard crystallisation process. On the left-hand side of Figure 1 nanoparticles 10 are represented by spheres and crystal forming material 12 are represented by cubes. On the left-hand side of this process the nanoparticles 10 and the crystal forming material 12 are dissolved in a 'good' solvent 14 which enables the crystal forming material 12 to form a solution. Crystallisation of the crystal forming material 12 occurs when a poor solvent 16 is added to the 'good' solvent 14. Crystallisation is shown on the right-hand side of Figure 1 wherein the crystal forming material 12 forms large crystal structures 18 with the nanoparticles 10 being suspended in the mixture formed by the 'good' solvent 14 and poor solvent 16.

Figure 2 is a representation of the coprecipitation according to the present invention. On the left-hand side of the coprecipitation process shown in Figure 2, nanoparticles 10 and crystal forming material 12 are shown to be dissolved in a 'good' solvent 14. On addition to a non-solvent 22, as shown in the right-hand side of the coprecipitation process, the crystal forming material 12 crystallises forming a larger crystal structure 20 and the nanoparticles 10 become coated to the outside surface of the crystal 20. The nanoparticles 10 are thought to be attached to the crystal 20 via, for example, van-der Waals interactions. The nanoparticles 10 are therefore non-covalently bonded.

Although not wishing to be bound by any theory, it is thought that the co-precipitation may proceed via an intermediate 24 wherein the nanoparticles 10 are initially included in the crystals 12 and are then forced thermodynamically to the outside surface of the formed crystal structure 20. The nanoparticle coated structure is formed in a single self-assembly step.

The formation of the nanoparticle coated crystals in a single coprecipitation step is an example of a single self-assembly process. Such self-assembly processes are recognised in the art as being the key to the so called bottom-up approach to forming nanostructured materials. In a self-assembly process each component that forms part of the material is tailored to have a particular chemical or physical property that promotes its organisation in the final material relative to the other components. In this case the component making up the crystal forming material is chosen to have a high crystal lattice energy so as to preferentially exclude nanoparticles from the interior of the crystal. The nanoparticle component is tailored to have solubility characteristics such that it is soluble in at least one solvent that the crystal forming material is also soluble in and insoluble in at least one non-solvent for the crystal-forming material, and wherein the solvent and non-solvent are susbstantially miscible or partially miscible.

The nanoparticle coated microcrystals generally exhibit three levels of self-assembly. Firstly, the nanoparticles and crystal forming material are phase-separated so that the nanoparticles are almost exclusively found on the outer surface of the core crystalline material rather than being included within the crystal lattice. Secondly, the nanoparticles are bound in a layer on the surface of the microcrystals rather than forming separate aggregates of nanoparticles. Thirdly, the nanoparticles may be ordered adjacent to each other on the surface of the microcrystals. This ordering is a function of:
i) the surface physical and chemical properties of the nanoparticles;
ii) the affinity of nanoparticles for binding to each other;
iii) the chemical structure and space group of the core crystal lattice;
iv) physical features on the surface of the crystal such as defects and steps; and
v) the affinity of the nanoparticles or clusters of nanoparticles for the crystal surface.

Changes to physical parameters or conditions which alter the properties of any of the above may change the sel-assembly process and change the ordering or extent of ordering. For example changes to the pH of the solution containing the mixture of nanoparticles, and crystal forming material will alter i), ii) and v).

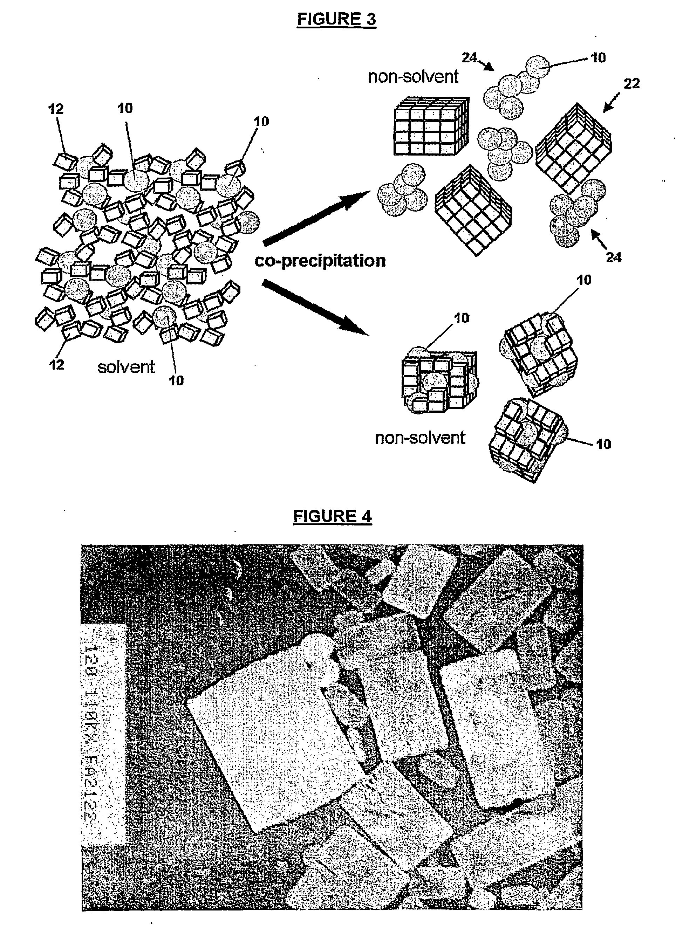

Figure 3 is a representation of two routes as to how the coprecipitation might have been expected to occur. On the left hand side of Figure 3, nanoparticles 10 and crystals 12 are shown to be dissolved in a 'good' solvent 14. On addition to a non-solvent 16 it might have been expected that the crystal forming material 12 would form large crystal structures 22 and the nanoparticles 10 form agglomerates 24. Alternatively, it might have been expected that the nanoparticles 10 would become included in a crystal structure formed by the crystal forming material 12. However, surprisingly this is not what is obtained.

Nanoparticles bound to the surface of microcrystals can be chemically modified. Modification of either the ligand bound to the surface or else the inner core material may be achieved. Chemical agents can be delivered through the gas-phase to a dry powder of the nanoparticle coated microcrystals or introduced into a solvent in which the microcrystals are suspended. This allows chemistry to be carried out on the nanoparticles in a solvent that they would not normally dissolve in. For example water soluble nanoparticles can be modified in a non-polar solvent with poorly water soluble compounds such as fluorescent dyes or therapeutic drugs. At least one side of the nanoparticle will be partly shielded from the chemical agent because it is in contact with the microcrystal. This also allows for nanoparticles with anisotropic properties to be produced because the surface more exposed to solvent will tend to be reacted more easily. This is particularly so when large bulky chemical agents are used. Following completion of a reaction and removal of excess chemical agents by rinsing the nanoparticle coated microcrystals, the microcrystals can be redissolved in another solvent. In this way nanoparticles can be bound temporarily onto the surface of microcrystals, chemically modified and then released by simple dissolution. Anisotropically modified nanoparticles produced by this route have many applications. For example, they can be used as surfactants, surface modifiers or as building blocks for self-assembly of more complex structures.

The driving force for the self-assembly process is the formation of the crystal lattice so therefore synthetic molecules with dimensions greater than the unit cell of the crystal lattice and with the right solubility characteristics will also undergo the same process. Preferably, the average diameter of the molecule in a compact conformation will be more than 50% larger than the minimum dimension of the unit cell of the crystal lattice formed by the coprecipitant. This will minimise inclusion of molecules into the types of crystals with high lattice energies that are preferred for the coprecipitation process. Molecules could be formed into nanostructures on their own or as mixtures with one or more other nanoparticles such as metals and semi-conductors. The molecules may have functional characteristics such as sensitivity to environmental changes such as solvent or light or else be therapeutic agents. In the latter case the high surface area and resultant high solubility of the particles will provide benefits in drug delivery applications. Another use of the molecules is to provide spacer units of defined dimension between nanoparticles on the surface of the microcrystals. Examples of suitable spacer units are synthetic DNA or helical peptides of well defined length, or dendrimers of a specific generation. Inclusion of spacer units can be used to increase the complexity of the structures formed on the surface of the microcrystals. Following formation of the mixed nanostructures the molecular spacer units can be used to permanently link two or more nanoparticles together. This can be achieved by chemical activation of groups on either the molecule or nanoparticle or else by introduction of cross-linking agents. The activation or cross-linking can be carried out on a suspension of the microcrystals in a solvent or as a dry powder. Coated microcrystals formed with a combination of metal nanoparticles and therapeutic molecules provides a route for immobilising therapeutic agents on high density particles for drug delivery by routes such as needleless injection. Alternatively, nanoparticle coated microcrystals may be formed by coprecipitation of a crystalline drug material with nanoparticles. If high density nanoparticles such as gold are used this provides a route for increasing the overall density of the drug-particle formed. High density drug particles are useful for-needleless injections.

Nanoparticle coated microcrystals have very high surface areas. Metal nanoparticles can be used for catalysing many reactions. One example is the growth of carbon nanotubes. These can be grown out from the surface of metal nanoparticles with the diameter of the nanoparticle defining the type and dimensions of carbon nanotube formed. Arrays of metal nanoparticles can be used to grow arrays of carbon nanotubes. Such aligned nanotubes could find applications in display applications with the dimensions of the microcrystals defining the area of the element. Mixtures of reactive and inert metal nanoparticles could be used that on fusion would provide non-reactive contacts to the carbon nanotubes grown from the reactive centres.

### Examples

### Example 1: Preparation of Gold Colloid

Gold nanoparticles soluble in water but insoluble in a range of water miscible solvents were first of all prepared.

0.155g of tetrachloroauric acid (HAuCl₄) is dissolved in 8.8ml of 6:1 methanol/acetic acid. 0.19 of N-(2-mercapto-propionyl)glycine (ie. tiopronin) was dissolved separately in another 8.8ml of 6:1 methanol/acetic acid. The tiopronin crystal-forming solution was added quickly to the HAuCl₄ solution with good stirring. The solution was allowed to mix for approximately 20 - 25 seconds during which time the colour changed slowly from light yellow to a deeper orange. After this a pre-prepared solution of KBH₄ (0.432g in 7.5 ml pure water) non-solvent was added quickly with stirring.

The suspension was allowed to cool, transferred into centrifuge tubes and repeatedly washed with and spun-down in methanol to remove all organic soluble impurities. The resulting dark nanoparticle powder is then removed from the flask with a spatula and dried in a vacuum desiccator overnight. UV spectra of an aqueous solution showed a maximum at ~520 nm which is an indicative of nanoparticles with an average core diameter of 4±2 nm. Transmission electron microscope images confirmed this.

### Example 2: Preparation of Gold Coated Water Soluble Crystals

The technique given below is typical of those used to prepare crystals of K₂SO₄ coated with the water soluble gold nanoparticles described above.

A K₂SO₄ gold nanoparticle mixture was prepared by adding 0.01 g of the gold nanoparticle powder to 1000 µl of a saturated aqueous solution of K₂SO₄. 200 µl of the resultant solution was then added to 3 ml of dry acetonitrile non-solvent with vigorous shaking. Immediately, a light grey precipitate was formed which settled freely on standing. The precipitate was then collected by decanting off the acetonitrile and placed in a vacuum desiccator overnight.

On admixing the aqueous mixture of gold nanoparticles and saturated K₂SO₄ with acetonitrile both components simultaneously coprecipitate. This is because they are both soluble in water but only sparingly soluble in acetonitrile. The simultaneous coprecipitation leads to the formation of nanoparticle coated microcrystals. The K₂SO₄ is able to rapidly nucleate and form microcrystals and the nanoparticles are excluded from the interior of these by the high lattice energy. Since the nanoparticles are insoluble in the solvent they form a layer over the surface of the crystals. Very surprisingly the nanoparticles in this layer are able to form organised arrays and characteristic patterns of lines arranged at similar angles to each are observed on many crystals. This indicates the underlying crystal lattice is able to influence the organisation of the nanoparticles in a process similar to epitaxial growth.

Figure 4 is a first representation of gold nanoparticles coated on to crystals of K₂SO₄.

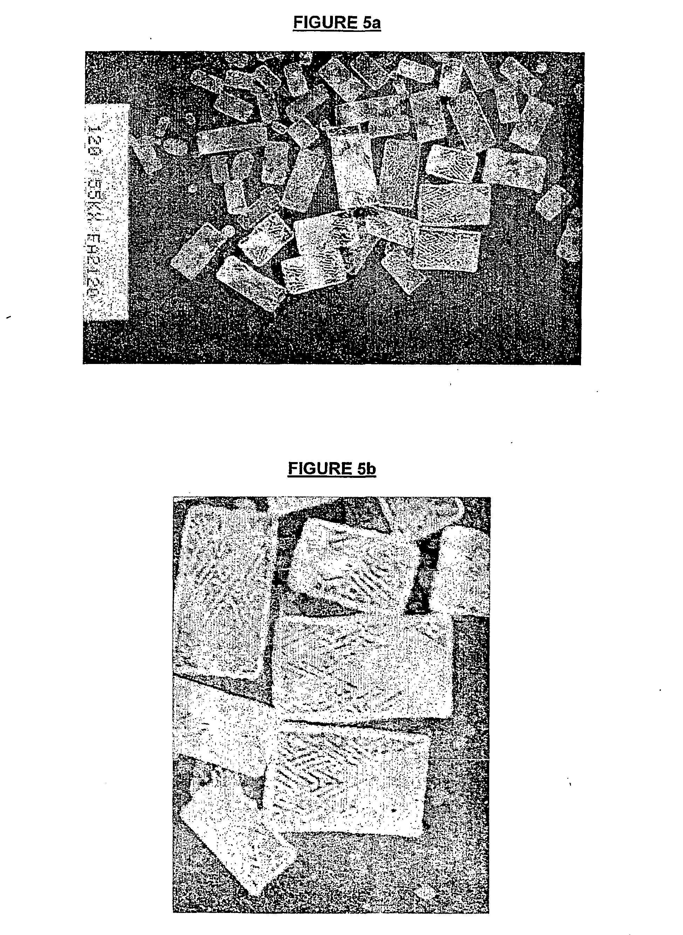

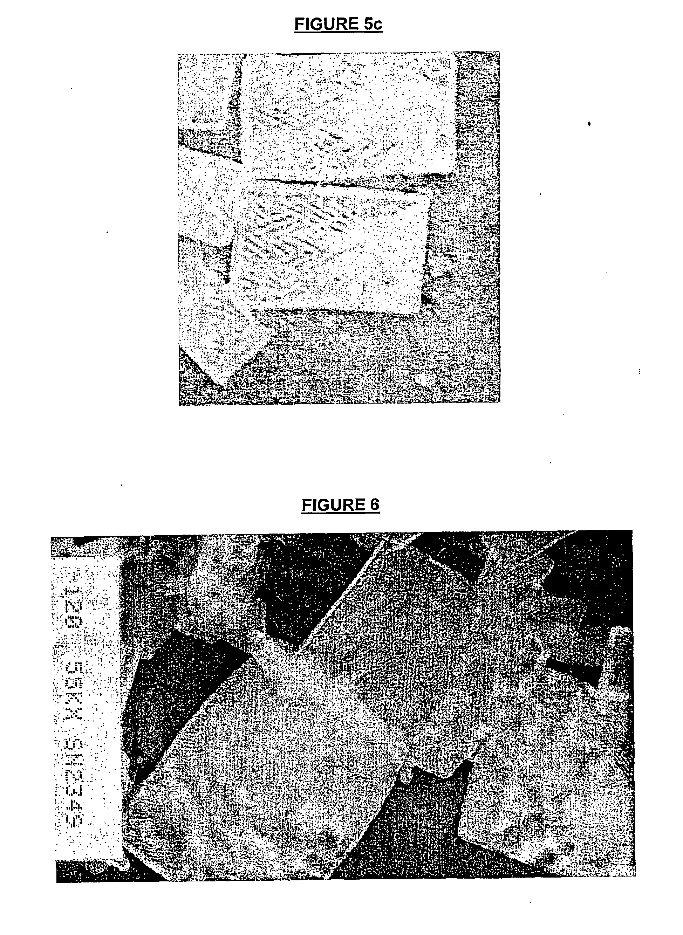

As shown in Figure 5a, the nanoparticles form a coating on the surface of the microcrystals. As expected more intense contrast is seen around the edges of the crystals where the beam is interrupted by nanoparticles that lie on the whole of the surfaces that run parallel to the beam. Figure 5b which is an expanded view of Figure 5a shows that the nanoparticles form a characteristic pattern of lines across the surface. This indicates that rather than a random coating of nanoparticles on the crystal surface, the coating is assembled in an ordered manner. Figure 5c is a further expanded view.

Using the presently described process of the order of 10¹² to 10¹⁵ nanoparticle coated crystals can be simultaneously made using about 1 litre of reaction mixture consisting of the metal colloid and the salt solution. About 10 - 10000 m² of crystal surface can be coated with nanoparticles using about 1 litre of reaction mixture.

Figure 6 shows that larger crystals of K₂SO₄ may also be coated with a patterned array of gold nanoparticles.

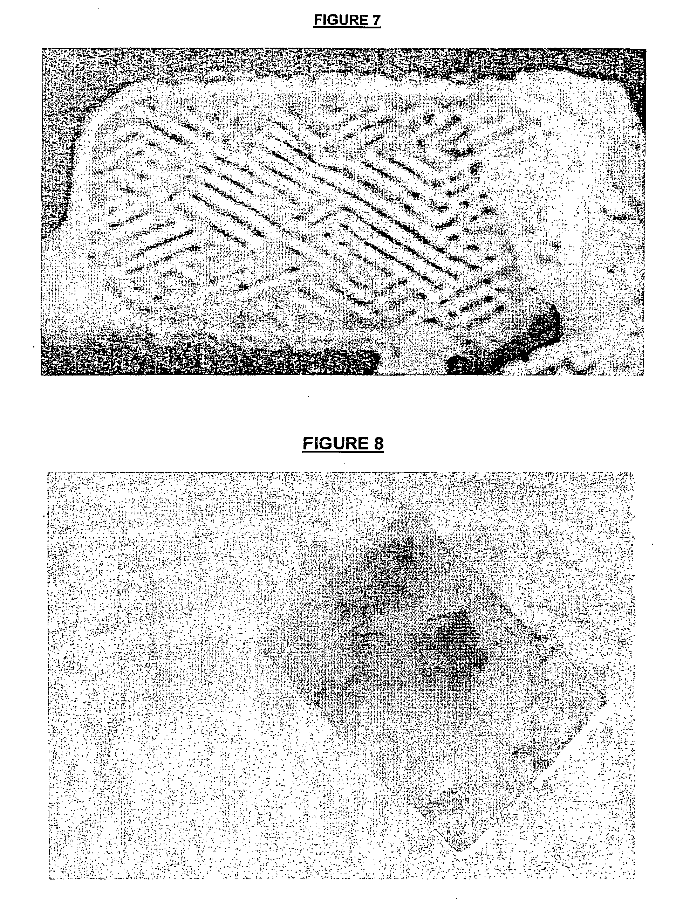

Figure 7 is a transmission electron microscope of a single gold nanoparticle K₂SO₄ crystal.

X-ray powder diffraction data showed that the nanoparticles on the gold surface did not disrupt and distort the crystal lattice. X-ray powder diffraction results showed that K₂SO₄ precipitated with and without the nanoparticle have the same arcanite structure. Table 1 below shows results of K₂SO₄ crystals with and without nanoparticle.

**Table 1 Refined unit cell parameters of the two powder samples, the form is Arcanite, which has space group Pmcn.**

| | | K₂SO₄ | K₂SO_{4 +} nanoparticles |
|---|---|---|---|
| | | | |
| a0 | | 0.57649 (4) nm | 0.57626 (8) nm |
| b0 | | 1.00444 (4) nm | 1.00420 (9) nm |
| c0 | | 0.74551 (3) nm | 0.74522 (7) nm |

### Example 3: Fusion of Gold Nanoparticles on the Crystal Surface

The ordered arrays produced are suitable for further processing. For instance it is possible to fuse the lines of gold nanoparticles together on the microcrystal surface to form nanowires.

Differential scanning calorimetry of a sample of gold nanoparticles prepared as described in Example 1 showed an exotherm at ~210°C. This is consistent with interparticle fusing leading to an overall lower surface area.

Around 100 mg of the dull grey nanoparticle coated crystals prepared as described in Example 1 were mixed with a twenty fold weight excess of pure K₂SO₄ microcrystals precipitated using the same procedure. This dilution process was undertaken to minimise the possibility of inter-crystal fusion. The mixed powder was transferred to a ceramic crucible and placed in a furnace under a slow nitrogen flow. The temperature was raised at 10°C per minute up to a final temperature of 330°C whereupon the sample was left to cool for 3 hours in the nitrogen flow. On inspection the heated powder could be seen to have changed colour from dull grey to lilac indicating a significant change to the gold nanoparticles had occurred.

As shown in Figure 8 TEM of the fused crystals showed that the same type of pattern of lines was retained. On placing the fused crystals into water the salt core immediately dissolved.

### Example 4: Preparation of Gold Nanoparticle Coated Valine Microcrystals

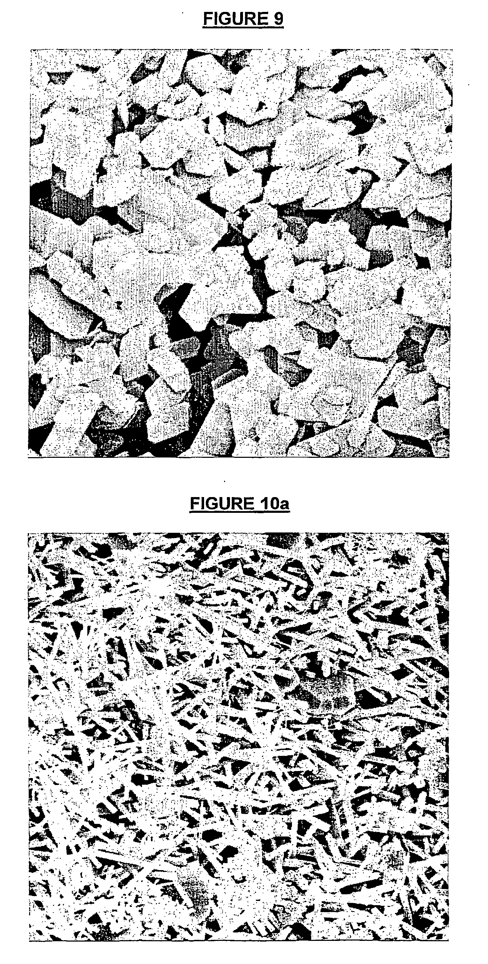

Gold nanoparticles (AuNP) were prepared according to the technique described in Example 1. A saturated aqueous solution of DL-Valine was prepared at room temperature and passed through a 20 micron filter to remove any undissolved material. A weighed sample of the gold nanoparticles was dissolved in the valine solution to give a concentration of AuNP of 1 mg /100 micro litres. 360 micro litres of this aqueous valine-AuNP mixture was then introduced dropwise and with rapid stirring to 15 ml of dry THF. Immediately upon addition, a grey precipitate of AuNP-valine microcrystals forms. The dark colour characteristic of the Au nanoparticles in the aqueous solution is fully associated with the microcrystals and can be recovered quantitatively on redissolution of the isolated microcrystals back into water. SEM images of the precipitate as shown in Figure 9 show regular plate-like valine microcrystals are formed with largest dimensions of less than 10 microns. No clusters of unbound nanoparticles can be seen. TEM is not possible because the gold coated valine microcrystals rapidly melt in the beam.

### Example 5: Preparation of Gold Nanoparticle coated Rb₂SO₄ Microcrystals

Gold nanoparticles (AuNP) were prepared according to the technique described in Example 1. A saturated aqueous solution of Rb₂SO₄ was prepared and passed through a 20 micron filter to remove any undissolved material. A weighed sample of the nanoparticles was dissolved in the saturated Rb₂SO₄ solution to give a concentration of AuNP of 1 mg /100 microlitres. 210 microlitres of this aqueous Rb₂SO₄-AuNP mixture was then introduced dropwise and with rapid stirring to 15 ml of dry DMF. Immediately upon addition, a grey precipitate of AuNP- Rb₂SO₄ microcrystals forms. The dark colour characteristic of the Au nanoparticles in the aqueous solution is fully associated with the microcrystals and can be recovered quantitatively on redissolution of the isolated microcrystals back into water.

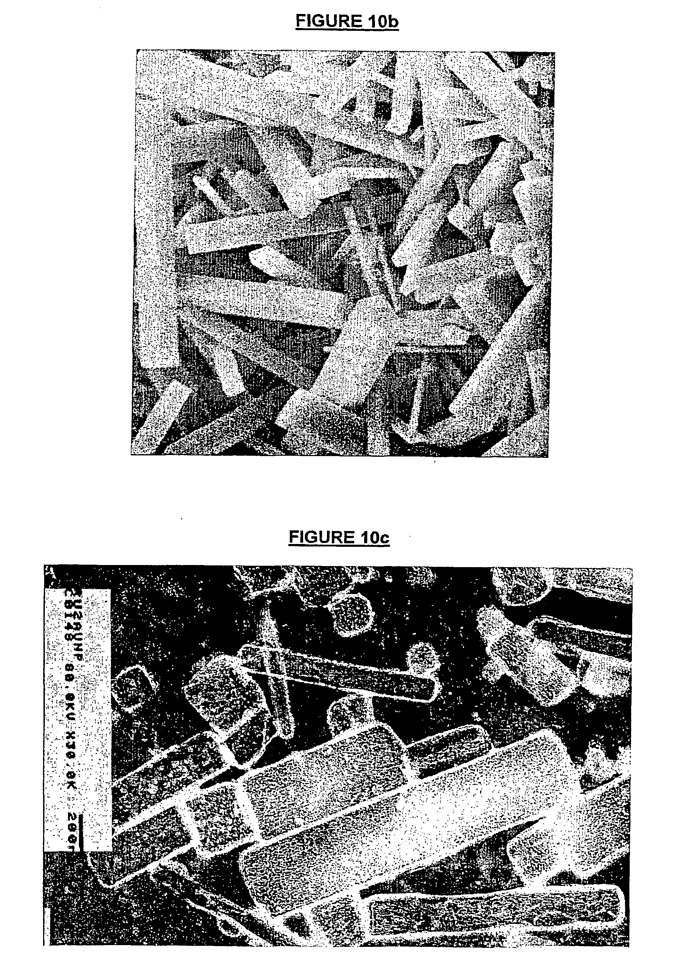

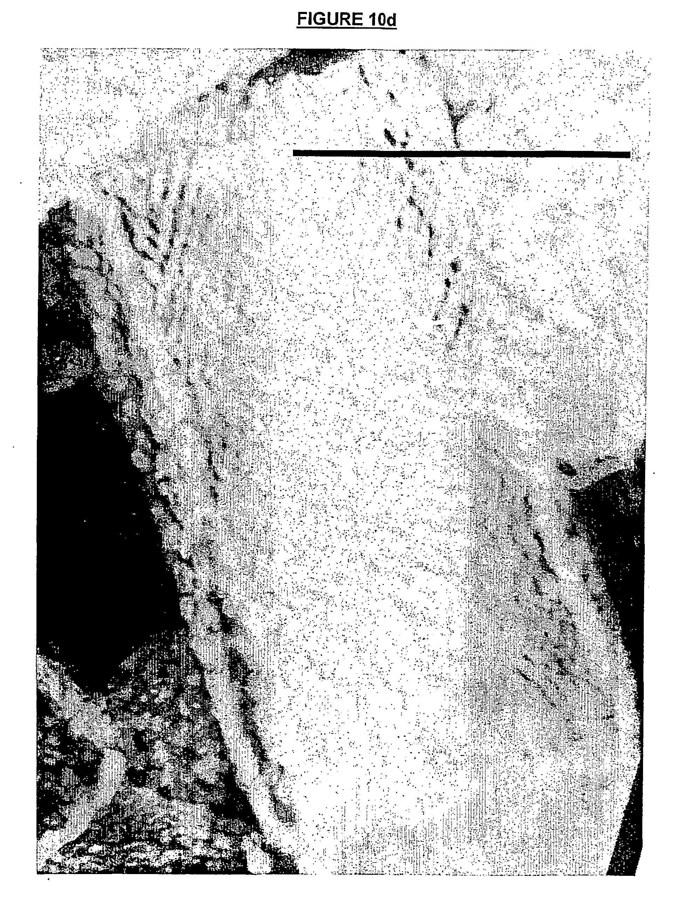

SEM images, as shown in Figures 10a and 10b, of the precipitate show regular (parallelpiped) shaped microcrystals are formed with largest dimensions of 10 microns. No clusters of unbound nanoparticles can be seen. Figure 10c is a TEM of the AuNP- Rb₂SO₄ microcrystals which shows the typical 'halos' expected when imaging a low contrast material (Rb₂SO₄) coated with the high contrast gold. Figure 10d is an expanded view of Figure 10c. At lower surface coverage it is possible to observe line features and patterns formed by the nanoparticles similar to those seen for K₂SO₄. The TEM of microcrystals prepared with high ratios of AuNP:Rb₂SO₄ (e.g. 3 mg/ 100 microlitre) give more densely coated microcrystals.

### Example 6: Production of Dendrimer Coated Microcrystals Using Starburst (Registered Trade Mark) PAMAM Generation 4

Starburst (Registered Trade Mark) (PAMAM) Dendrimer Generation 4 (amine terminated) and Starburst (Registered Trade Mark) PAMAM Dendrimer Generation 4.5 (carboxyl terminated) were obtained from Aldrich Chemicals as solutions and samples evaporated to dryness as required.

17.2 mg of dried Starburst (Registered Trade Mark) PAMAM Generation 4 dendrimer was dissolved in 860 micro litres of saturated K₂SO₄ solution. The sample was sonicated for 5 minutes to ensure that all the dendrimer was dissolved. Using a 250 microlitre syringe and vigorous stirring, the dendrimer/salt solution was then added dropwise to 2 lots of 15 ml of isopropanol (half the solution being added to each 15ml of solvent). Immediately upon addition, a fine white precipitate of microcrystals was formed.

To establish that the dendrimer was indeed present on the surface of the crystals, one of the samples was taken and put into suspension in isopropanol. To the suspension approximately 10 drops of an amine specific test reagent, trinitrobenzene sulfonic acid (TNBS), was added. As the acid was added to the sample, the microcrystals turned a bright orange/red colour clearly indicating reaction of TNBS with the free-amines on the surface of the dendrimer located on the microcrystals. The suspension was briefly centrifuged to settle the crystals. No positive amine test was observed in the solvent.

### Example 7: Production of Dendrimer Coated Microcrystals Using Starburst (Registered Trade Mark) PAMAM Generation 4.5

When the experiment in Example 6 was repeated using Starburst (Registered Trade Mark) PAMAM Generation 4.5 dendrimer (which has carboxylate rather than amino end groups), no orange colour was observed, proving that the positive amine test could only be attributed to the Starburst (Registered Trade Mark) PAMAM Generation 4 dendrimer used in the first experiment and not to any other species in the system.

Together the results shown in Examples 6 and 7 firmly establish that following coprecipitation the dendrimer is intimately associated with the microcrystals. In order to prove that the Starburst (Registered Trade Mark) PAMAM Generation 4.0 and 4.5 is located on the surface of the microcrystals a further test was carried out. The suspension was centrifuged and the isopropanol solvent decanted off, leaving a plug of orange crystals. To this was added 2 ml of saturated aqueous K₂SO₄ with vigorous shaking. On standing for 2 to 3 minutes very pale yellow K₂SO₄ crystals precipitate to the bottom of the vial leaving a clear orange/red coloured supernatant. This result demonstrates that the dendrimers were indeed situated on the surface of the crystal since they readily dissolve into the aqueous solution while the salt microcrystals (insoluble in saturated aqueous K₂SO₄) are left unaffected.

Examples 6 and 7 demonstrate formation of microcrystals coated with nanoparticles that have an insulating core material and positive outer surface.

### Example 8: Formation of Microcrystals Coated with a Mixture of Gold Nanoparticles and PAMAM Generation 4.5 Dendrimer

In this experiment, a mixture of two different types of nanoparticles were dissolved in a salt solution and all three coprecipitated.

Gold nanoparticles as prepared in Example 1 and Starburst (Registered Trade Mark) PAMAM Generation 4.5 dendrimer were dissolved in a saturated K₂SO₄ solution (9.9 mg of dendrimer and 4.2mg of AuNPs were dissolved in 1410 micro litres of K₂SO₄ solution). A clear dark solution identical to that obtained with gold nanoparticles in Example 2 resulted.

The sample was added to 15 ml isopropanol as described in Example 2. Upon addition to the isopropanol a grey/blue precipitate of microcrystals was observed immediately. The colour of these microcrystals is different from previously observed for either the individual dendrimer or AuNP experiments and clearly shows the nanoparticles are bound to the microcrystal in a different environment. The addition of trinitrobenzene sulfonic acid to the suspension turned the crystals a dark orange colour showing the amine containing PAMAM dendrimer is also present on the crystal surface.

This Example demonstrates formation of microcrystals coated with a mixture of two different types of nanoparticles; one type insulating (positive surface) and the other conducting(negative surface).

### Example 9: Preparation of Nanoparticle Array on Microcrystal Surface

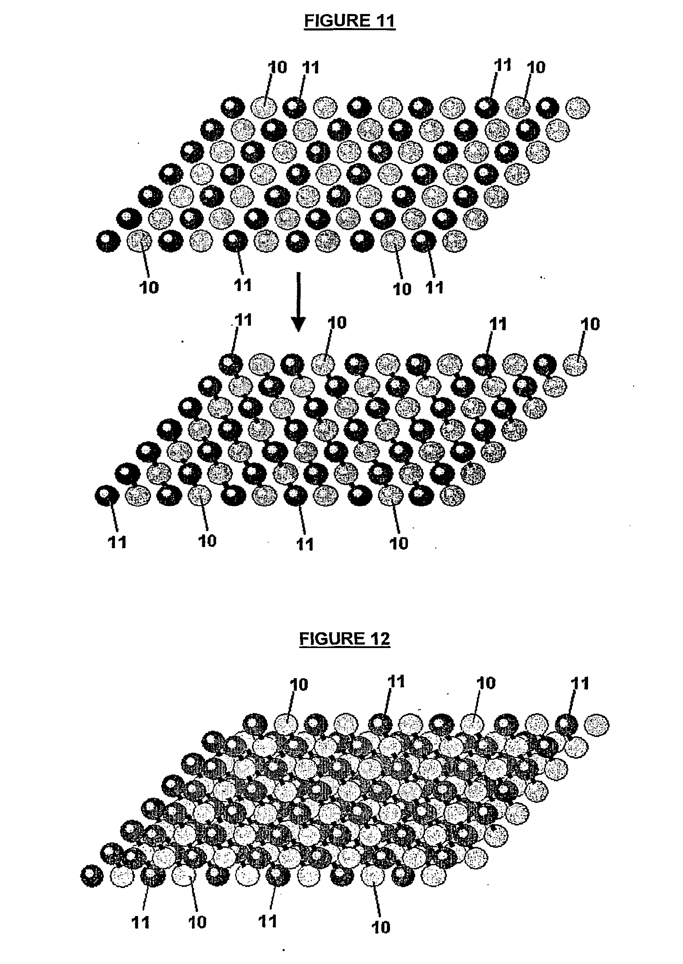

Figure 11 is a schematic representation of two different types of nanoparticles 10, 11 which are coated onto a crystal 20. The nanoparticles 10, 11 are then cross-linked using either a bifunctional molecules such as or an activating agent. The cross-linked structure may be used as a template to build subsequent layers as shown in Figure 12 which is a representation of a bi-layer of nanoparticles 10, 11.

### Example 10: Preparation of Anisotropic Nanoparticles

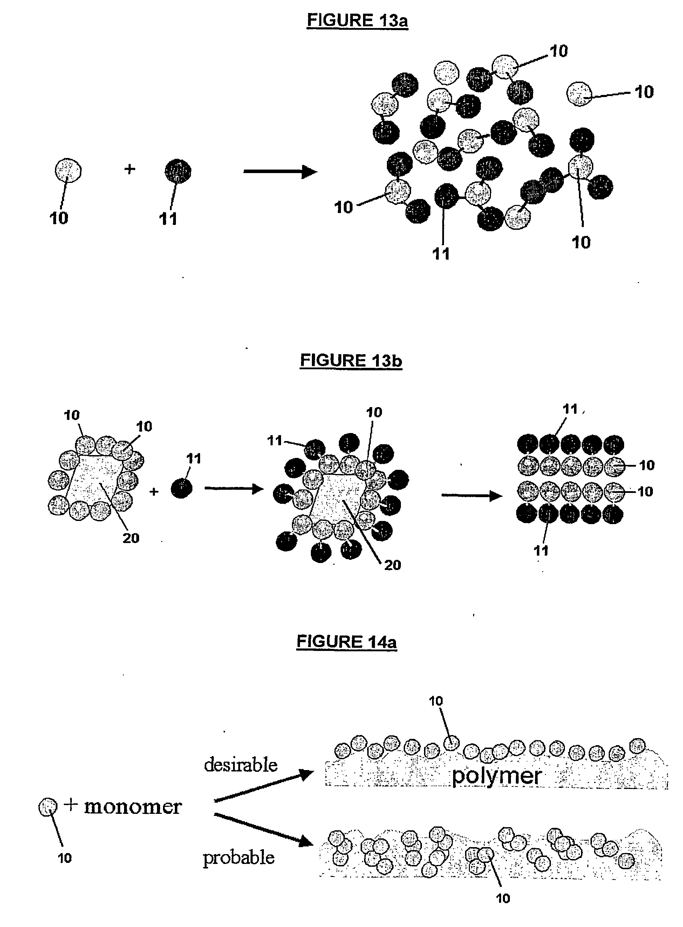

Figure 13a is a representation of the standard reaction of two different nanoparticles 10, 11 whereupon on reaction a variety of different combinations of the nanoparticles 10, 11 are formed. There is no selectivity in this reaction.

Figure 13b represents a method of selectively forming regular anisotropic nanoparticles. First of all, as described previously, nanoparticles 10 are coated on the surface of a crystal 20. An excess of a second nanoparticle 11 is then added to a solution of the nanoparticles 10 coated on the surface of the crystal 20. On addition of nanoparticles 11, the nanoparticles 11 covalently attach themselves to one face of the nanoparticles 10. On rinsing off excess reagents and redissolving the crystal 20 well-defined dimensions of nanoparticles 10 covalently bind to nanoparticles 11.

Regular anisotropic particles formed according to this method may be used for example, surfactants, surface modifiers or as building blocks for self-assembly of more complex structures.

### Example 11: Forming Nanoparticles at a Polymer Surface

Figure 14a is a representation of the standard reaction of a nanoparticle 10 with a monomer. Although, as shown in Figure 14a it is desirable for the nanoparticles 10 to form on top of a formed polymer substrate 30, the nanoparticles 10 usually incorporate themselves into the polymer substrate 30.

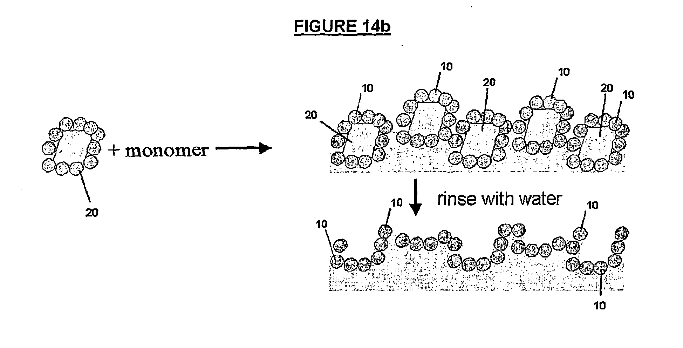

However, Figure 14b shows that if we use nanoparticles 10 coated onto the surface of a crystal 20 according to the present invention and mix this with the monomer mixture, following polymerisation the crystal will be bound onto the polymer. On rinsing, the crystals 20 dissolve and the nanoparticles are necessarily exposed at the surface of a void left by the loss of the crystal. This void may be on the exterior or interior of the polymer.

By forming the nanoparticles 10 at the surface of the polymer substrate 30, a very high surface area of the nanoparticles 10 is formed which is useful for catalysis, chromatography, diagnostics, separation technology, and analysis.

## Claims

1. A method of preparing nanoparticle coated crystals comprising the steps of:
(a) providing a mixture comprising nanoparticles and a solution of a crystal forming material; and
(b) coprecipitating the nanoparticles and the crystal forming material such that crystals are formed, a surface or surfaces of which are at least partially coated with nanoparticles; and
wherein the nanoparticles are provided as a dispersion or solution and are comprised of one of or a combination of any of the following: metals, metal alloys, semi-metals, semi-conductors, carbon allotropes, insulators and mixtures thereof.

2. A method of preparing nanoparticle coated crystals according to claim 1, wherein the carbon allotropes are any of the following: fullerenes, C₆₀ or carbon nanotubes, and mixtures thereof.

3. A method of preparing nanoparticle coated crystals according to any of claims 1 or 2 wherein the coprecipitation method makes use of a non-solvent.

4. A method of preparing nanoparticle coated crystals according to any preceding claim wherein the nanoparticles have a cross-section of about 0.5-250 nm, 1-20 nm or about 4 nm with a size distribution of about a mean value of 200% or ± 50%.

5. A method of preparing nanoparticle coated crystals according to any preceding claim, wherein the nanoparticles in solution have a monolayer coating on their outer surface which stabilises the nanoparticles.

6. A method of preparing nanoparticle coated crystals according to any of claims 3 to 5 wherein a solvent in which the crystal forming material and nanoparticles are dissolved in together is fully or partially miscible with the non-solvent used in the coprecipitation.

7. A method of preparing nanoparticle coated crystals according to any preceding claim wherein the nanoparticles form a coating on the crystal in the form of any of the following: a close-packed assembly of nanoparticles, open-structures such as in the form of a patterned array, and 2-dimensional or 3-dimensional structures.

8. A method of preparing nanoparticle coated crystals according to any preceding claim wherein the nanoparticle coating is in the form of a sub-monolayer, monolayer, a bilayer or a multilayer.

9. A method of preparing nanoparticle coated crystals according to any preceding claim wherein the percentage of surface coverage on one or more of the crystal surfaces is selected from any of the following: 1-100%, 20-80%, or 40-60%.

10. A method of preparing nanoparticle coated crystals according to any preceding claim wherein groups of adjacent nanoparticles on the crystal surface are organised relative to each other such as into lines, parallel lines, intersecting lines and lines that form fixed angles relative to each other.

11. A method of preparing nanoparticle coated crystals according to any preceding claim wherein lines of nanoparticles have a width of 0.5-100 nm and/or a height of 0.5-100 nm and/or lengths of 0.5-5000 nm.

12. A method of preparing nanoparticle coated crystals according to any preceding claim wherein the nanoparticles are formed from one of or a combination of any of the following: gold, silver, platinum, palladium, cobalt, rubidium, and alloys thereof.

13. A method of preparing nanoparticle coated crystals according to any preceding claim wherein two or more different types of nanoparticles are used to form the nanoparticle coating.

14. A method of preparing nanoparticle coated crystals according to claim 1, wherein the insulators are in the form of organic or inorganic dendrimers or hyperbranched polymers.

15. A method of preparing nanoparticle coated crystals according to any preceding claim wherein the crystals are selected from any of the following: water-soluble ionic materials such as an inorganic salt of KCl, K₂SO₄; organic solvent soluble ionic salts such as LiClO₄; highly polar or ionic compounds such as zwitterions of amino acids; organic salts such as sodium glutamate; sugars such as lactose; and other high melting point molecules such as pharmaceutical agents such as drugs, heterocycles and H-bonding molecules.

16. A method of preparing nanoparticle coated crystals according to any preceding claim wherein the crystal forming material is provided as a substantially saturated or near-saturated or highly concentrated solution.

17. A method of preparing nanoparticle coated crystals according to any preceding claim wherein the crystals are of a nanometre-micrometre dimension such as in the order of 5 nm - 100 µm or 25 nm - 10 µm.

18. A method of preparing nanoparticle coated crystals according to any preceding claim wherein adjacent nanoparticles are fused together by heating.

19. A method of preparing nanoparticle coated crystals according to any of claims 1 to 17 wherein adjacent nanoparticles are cross-linked by chemical means.

20. A method of preparing nanoparticle coated crystals according to any of claims 18 or 19 wherein after fusing or cross-linking the nanoparticles together, the crystal is dissolved by placing in an appropriate solvent, so as to leave behind hollow structures of fused nanoparticles, such as wire or tube-like structures, sheets, lattices or boxes.

21. A method of preparing nanoparticle coated crystals according to claims 2 to 20 wherein the non-solvent is selected from any of the following: organic liquids comprising of polar solvents (e.g. ethanol, propanol, acetone, acetonitrile, dimethylformamide), intermediate solvents (e.g. ethyl acetate, tetrahydrofuran) or non-polar solvents (e.g. toluene, hexane) and mixtures thereof, near-critical and super-critical fluids (e.g. carbon dioxide) and/or acids and bases such as aqueous acids (e.g. HCl (aq)), organic bases (NaOH), organic acids (e.g. acetic acid) and organic bases (e.g. pyridine).

22. A method of preparing nanoparticle coated crystals according to any preceding claim wherein the nanoparticles coated on the surface of the crystals are accessible for modification by chemical, biochemical or photochemical reactions.

23. A method of preparing nanoparticle coated crystals according to any preceding claim wherein the coated nanoparticles act as templates for organisation of secondary layers so as to form a bilayer or multilayer of nanoparticles or molecules as absorbed from solution or the gas phase.

24. A method of preparing nanoparticle coated crystals according to any preceding claim wherein during the coating process functional molecules are also attached to the crystal.

## Patentansprüche

1. Verfahren zum Herstellen von mit Nanopartikeln beschichteten Kristallen, welches die nachfolgenden Verfahrensschritte aufweist:
(a) Bereitstellung einer Mischung, welche Nanopartikel und eine Lösung eines Kristalle bildenden Materials enthält; und
(b) ein gemeinsames Niederschlagen der Nanopartikel und des Kristalle bildenden Materials derart, dass Kristalle gebildet werden, von denen eine Oberfläche oder Oberflächen mindestens teilweise mit Nanopartikeln beschichtet sind; und
wobei die Nanopartikel als eine Dispersion oder als eine Lösung bereitgestellt werden und aus einem oder aus einer Kombination von irgendwelchen der nachfolgenden Stoffe bestehen: Metalle, Metalllegierungen, Halbmetalle, Halbleiter, Kohlenstoffallotrope, Isolatoren und Mischungen derselben.

2. Verfahren zum Herstellen von mit Nanopartikeln beschichteten Kristallen gemäß Anspruch 1, bei welchem die Kohlenstoffallotrope aus irgendeinem der folgenden Stoffe bestehen: Fullerene, C₆₀ oder Kolilenstoffnanorohre und Mischungen derselben.

3. Verfahren zum Herstellen von mit Nanopartikeln beschichteten Kristallen gemäß irgendeinem der Ansprüche 1 oder 2, bei welchem das Verfahren des gemeinsamen Niederschlagens von einem Nicht-Lösungsmittel Gebrauch macht.

4. Verfahren zum Herstellen von mit Nanopartikeln beschichteten Kristallen gemäß irgendeinem der vorhergehenden Ansprüche, bei welchem die Nanopartikel einen Querschnitt von etwa 0,5-250 nm, 1-20 nm oder etwa 4 nm aufweisen, mit einer Größenveiteilung von etwa einem Mittelwert von 200 % oder ±50 %.

5. Verfahren zum Herstellen von mit Nanopartikeln beschichteten Kristallen gemäß irgendeinem der vorhergehenden Ansprüche, bei welchem die Nanopartikel in Lösung eine einlagige Beschichtung auf ihrer äußeren Oberfläche aufweisen, welche die Nanopartikel stabilisiert.

6. Verfahren zum Herstellen von mit Nanopartikeln beschichteten Kristallen gemäß irgendeinem der Ansprüche 3 bis 5, bei welchem ein Lösungsmittel, in dem das Kristalle bildende Material und Nanopartikel zusammen aufgelöst sind, vollständig oder teilweise mit dem Nicht-Lösungsmittel mischbar ist, welches bei dem gemeinsamen Niederschlagen verwendet wird.

7. Verfahren zum Herstellen von mit Nanopartikeln beschichteten Kristallen gemäß irgendeinem der vorhergehenden Ansprüche, bei welchem die Nanopartikel auf dem Kristall eine Beschichtung von irgendeiner der nachfolgenden Formen bilden: eng gepackte Anordnung von Nanopartikeln, offene Strukturen wie etwa in der Form einer gemusterten Anordnung und 2-dimensionale oder 3-dimensionale Strukturen.

8. Verfahren zum Herstellen von mit Nanopartikeln beschichteten Kristallen gemäß irgendeinem der vorhergehenden Ansprüche, bei welchem die Nanopartilcelbeschichtung vorliegt in der Form einer subeinlagigen, einlagigen, zweilagigen oder mehrlagigen Beschichtung.

9. Verfahren zum Herstellen von mit Nanopartikeln beschichteten Kristallen gemäß irgendeinem der vorhergehenden Ansprüche, bei welchem der Prozentsatz der Oberflächenbedeckung auf einer oder auf mehreren der Kristalloberflächen aus irgendeinem der folgenden Werte ausgewählt wird: 1-100%, 20-80 % oder 40-60 %.

10. Verfahren zum Herstellen von mit Nanopartikeln beschichteten Kristallen gemäß irgendeinem der vorhergehenden Ansprüche, bei welchem Gruppen von benachbarten Nanopartikeln auf der Kristalloberfläche relativ zueinander organisiert sind wie etwa in Linien, in parallelen Linien, in sich schneidenden Linien und in Linienanordnungen, die feste Winkel relativ zueinander bilden.

11. Verfahren zum Herstellen von mit Nanopartikcln beschicliteten Kristallen gemäß irgendeinem der vorhergehenden Ansprüche, bei welchem Linien von Nanopartikeln eine Breite von 0,5-100 nm und / oder eine Höhe von 0,5-100 nm und / oder eine Längen von 0,5-5000 nm aufweisen.

12. Verfahren zum Herstellen von mit Nanopartikeln beschichteten Kristallen gemäß irgendeinem der vorhergehenden Ansprüche, bei welchem die Nanopartikel aus einem oder aus einer Kombination von irgendeinem der nachfolgenden Stoffe hergestellt werden: Gold, Silber, Platin, Palladium, Cobalt, Rubidium und Legierungen derselben.

13. Verfahren zum Herstellen von mit Nanopartikeln beschichteten Kristallen gemäß irgendeinem der vorhergehenden Ansprüche, bei welchem zwei oder mehrere verschiedene Typen von Nanopartikeln verwendet werden, um die Nanopartikelbeschichtung herzustellen.

14. Verfahren zum Herstellen von mit Nanopartikeln beschichteten Kristallen gemäß Anspruch 1, bei welchem die Isolatoren in der Form von organischen oder anorganischen Dendrimeren oder von hyperverzwcigten Polymeren vorliegen.

15. Verfahren zum Herstellen von mit Nanopartikeln beschichteten Kristallen gemäß irgendeinem der vorhergehenden Ansprüche, bei welchem die Kristalle ausgewählt werden unter irgendeinem der nachfolgenden Stoffe: wasserlösliche ionische Materialien wie etwa ein anorganisches Salz von KCL, K₂SO₄; in organischen Lösungsmitteln lösliche ionische Salze wie etwa LiClO₄; hochpolare oder ionische Verbindungen wie etwa Zwitterionen von Aminosäuren; organische Salze wie etwa Natriumglutamat; Zucker wie etwa Lactose; und andere Moleküle mit einem hohen Schmelzpunkt wie etwa pharmazeutische Agenzien wie etwa Arzneimittel, Heterozyklische und H bindende Moleküle.

16. Verfahren zum Herstellen von mit Nanopartikeln beschichteten Kristallen gemäß irgendeinem der vorhergehenden Ansprüche, bei welchem das Kristalle bildende Material als eine im Wesentlichen gesättigte oder nahezu gesättigte oder hoch konzentrierte Lösung bereitgestellt wird.

17. Verfahren zum Herstellen von mit Nanopartikeln beschichteten Kristallen gemäß irgendeinem der vorhergehenden Ansprüche, bei welchem die Kristalle in dem Dimensionsbereich von Nanometem-Mikrometern vorliegen wie etwa in der Größenordnung von 5 um - 100 µm oder 25 nm - 10 µm.

18. Verfahren zum Herstellen von mit Nanopartikeln beschichteten Kristallen gemäß irgendeinem der vorhergehenden Ansprüche, bei welchem die benachbarten Nanopartikel durch ein Erwärmen zusammengeschmolzen werden.

19. Verfahren zum Herstellen von mit Nanopartikeln beschichteten Kristallen gemäß irgendeinem der Ansprüche 1 bis 17, bei welchem die benachbarten Nanopartikel durch chemische Mittel vernetzt werden.

20. Verfahren zum Herstellen von mit Nanopartikeln beschichteten Kristallen gemäß irgendeinem der Ansprüche 18 oder 19, bei welchem, nachdem die Nanopartikel zusammengeschmolzen oder zusammenvernetzt worden sind, der Kristall aufgelöst wird, indem man ihn in ein geeignetes Lösungsmittel tut, um so hohle Strukturen von geschmolzenen Nanopartikeln zurückzulassen, wie etwa draht- oder rohrähnliche Strukturen, Blätter, Gitter oder Kästen.

21. Verfahren zum Herstellen von mit Nanopartikeln beschichteten Kristallen gemäß den Ansprüchen 2 bis 20, bei welchem das Nicht-Lösungsmittel ausgewählt wird unter irgendeinem der folgenden Mittel: organische Flüssigkeiten, welche polare Lösungsmittel umfassen (z.B.Ethanol, Propanol, Aceton, Acetonitril, Dimethylformamid), Zwischenlösungsmittel (z.B. Ethylacetat, Tetrahydrofuran) oder nicht polare Lösungsmittel (z.B. Toluol, Hexan) und Mischungen derselben, nahezu kritische und superkritische Fluide (z.B. Kohlenstoffdioxid) und / oder Säuren und Basen wie etwa wässrige Säuren [z.B. HCL (aq.), anorganische Basen (NaOH), organische Säuren (z.B. Essigsäure) und organische Basen (z.B. Pyridin).

22. Verfahren zum Herstellen von mit Nanopartikeln beschichteten Kristallen gemäß irgendeinem der vorhergehenden Ansprüche, bei welchem die Nanopartikel, die auf die Oberfläche der Kristalle beschichtet worden sind, zugänglich sind für eine Modifikation durch chemische, biochemische oder photochemische Reaktionen.

23. Verfahren zum Herstellen von mit Nanopartikeln beschichteten Kristallen gemäß irgendeinem der vorhergehenden Ansprüche, bei welchem die beschichteten Nanopartikel als Vorlagematrize für die Organisation von Sekundärlagen wirken, um so eine zweilagige oder mehrlagige Schicht von Nanopartikeln oder von Molekülen herzustellen, so wie dieselben aus der Lösung oder aus der Gasphase absorbiert werden.

24. Verfahren zum Herstellen von mit Alanopartikeln beschichteten Kristallen gemäß irgendeinem der vorhergehenden Ansprüclie, bei welchem während des Beschichtungsverfahrens auch funktionale Moleküle an den Kristall gebunden werden.

## Revendications

1. Procédé pour la préparation de cristaux revêtus de nanoparticules comprenant les étapes:
(a) de fourniture d'un mélange comprenant des nanoparticules et une solution d'un matériau formant des cristaux; et
(b) de co-précipitation des nanoparticules et du matériau formant des cristaux de sorte que des cristaux sont formés, dont une ou des surfaces sont au moins partiellement revêtues avec les nanoparticules; et
dans lequel les nanoparticules sont apportées sous forme d'une dispersion ou d'une solution et sont composées d'un élément ou d'une combinaison de n'importe quels éléments de ce qui suit: métaux, alliages métalliques, semi-métaux, semi-conducteurs, allotropes de carbone, isolants et mélanges de ceux-ci.

2. Procédé pour la préparation de cristaux revêtus de nanoparticules suivant la revendication 1, dans lequel les allotropes de carbone sont n'importe lesquels éléments de ce qui suit: fullerènes, C₆₀ ou nanotubes de carbone et mélanges de ceux-ci.

3. Procédé pour la préparation de cristaux revêtus de nanoparticules suivant l'une quelconque des revendications 1 et 2, dans lequel le procédé de co-précipitation fait usage d'un non solvant.

4. Procédé pour la préparation de cristaux revêtus de nanoparticules suivant l'une quelconque des revendications précédentes, dans lequel les nanoparticules possèdent une section transversale d'environ 0,5-250 nm, de 1-20 nm ou d'environ 4 nm avec une distribution de tailles d'environ une valeur moyenne de 200% ou ± 50%.

5. Procédé pour la préparation de cristaux revêtus de nanoparticules suivant l'une quelconque des revendications précédentes, dans lequel les nanoparticules en solution présentent un revêtement monocouche sur leur surface externe qui stabilise les nanoparticules.

6. Procédé pour la préparation de cristaux revêtus de nanoparticules suivant l'une quelconque des revendications 3 à 5, dans lequel un solvant où le matériau formant des cristaux et les nanoparticules sont dissous ensemble est totalement ou partiellement miscible avec le non solvant utilisé dans la co-précipitation.

7. Procédé pour la préparation de cristaux revêtus de nanoparticules suivant l'une quelconque des revendications précédentes, dans lequel les nanoparticules forment un revêtement sur le cristal sous la forme de n'importe lequel de ce qui suit: un ensemble très tassé de nanoparticules, des structures ouvertes telles que sous la forme d'un réseau à motif et des structures à 2 dimensions ou à 3 dimensions.

8. Procédé pour la préparation de cristaux revêtus de nanoparticules suivant l'une quelconque des revendications précédentes, dans lequel le revêtement de nanoparticules est sous la forme d'une sous-monocouche, d'une monocouche, d'une bicouche ou d'une multicouche.

9. Procédé pour la préparation de cristaux revêtus de nanoparticules suivant l'une quelconque des revendications précédentes, dans lequel le pourcentage de couverture de surface sur une ou plusieurs surfaces des cristaux est choisi parmi n'importe lequel de ce qui suit: 1-100%, 20-80% ou 40-60%.

10. Procédé pour la préparation de cristaux revêtus de nanoparticules suivant l'une quelconque des revendications précédentes, dans lequel des groupes de nanoparticules adjacentes sur la surface des cristaux sont organisés les uns par rapport aux autres comme en lignes, lignes parallèles, lignes qui se coupent et lignes qui forment des angles fixes les unes par rapport aux autres.

11. Procédé pour la préparation de cristaux revêtus de nanoparticules suivant l'une quelconque des revendications précédentes, dans lequel les lignes de nanoparticules ont une largeur de 0,5-100 nm et/ou une hauteur de 0,5-100 nm et/ou des longueurs de 0,5-5000 nm.

12. Procédé pour la préparation de cristaux revêtus de nanoparticules suivant l'une quelconque des revendications précédentes, dans lequel les nanoparticules sont formées d'un élément ou d'une combinaison de n'importe lesquels éléments de ce qui suit: or, argent, platine, palladium, cobalt, rubidium et alliages de ceux-ci.

13. Procédé pour la préparation de cristaux revêtus de nanoparticules suivant l'une quelconque des revendications précédentes, dans lequel deux types différents ou plus de nanoparticules sont utilisés pour former le revêtement de nanoparticules.

14. Procédé pour la préparation de cristaux revêtus de nanoparticules suivant la revendication 1, dans lequel les isolants sont sous la forme de dendrimères organiques ou inorganiques ou de polymères hyper-ramifiés.

15. Procédé pour la préparation de cristaux revêtus de nanoparticules suivant l'une quelconque des revendications précédentes, dans lequel les cristaux sont choisis parmi n'importe lesquels de ce qui suit: matériaux ioniques solubles dans l'eau tels qu'un sel inorganique de KCl, K₂SO₄; sels ioniques solubles dans un solvant organique tels que LiClO₄; composés hautement polaires ou ioniques tels que des zwittérions d'aminoacides; sels organiques tels que le glutamate de sodium; sucres tels que le lactose; et d'autres molécules à haut point de fusion telles que des agents pharmaceutiques comme des médicaments, des hétérocycles et des molécules à liaison H.

16. Procédé pour la préparation de cristaux revêtus de nanoparticules suivant l'une quelconque des revendications précédentes, dans lequel le matériau formant des cristaux est apporté sous forme d'une solution substantiellement saturée ou presque saturée ou fortement concentrée.

17. Procédé pour la préparation de cristaux revêtus de nanoparticules suivant l'une quelconque des revendications précédentes, dans lequel les cristaux sont d'une dimension nanomètre-mieromètre comme de l'ordre de 5 nm-100 µm ou 25 nm-10 µm.

18. Procédé pour la préparation de cristaux revêtus de nanoparticules suivant l'une quelconque des revendications précédentes, dans lequel des nanoparticules adjacentes sont fondues ensemble par un chauffage.

19. Procédé pour la préparation de cristaux revêtus de nanoparticules suivant l'une quelconque des revendications 1 à 17, dans lequel des nanoparticules adjacentes sont réticulées par un moyen chimique.

20. Procédé pour la préparation de cristaux revêtus de nanoparticules suivant l'une quelconque des revendications 18 et 19, dans lequel après la fusion ou la réticulation des nanoparticules ensemble, le cristal est dissous en le plaçant dans un solvant approprié, de manière à se débarrasser des structures creuses de nanoparticules fondues, telles que des structures de type fil ou tube, des feuilles, des treillis ou des boîtes.

21. Procédé pour la préparation de cristaux revêtus de nanoparticules suivant les revendications 2 à 20, dans lequel le non solvant est choisi parmi n'importe lesquels de ce qui suit: liquides organiques comprenant des solvants polaires (par ex. éthanol, propanol, acétone, acétonitrile, diméthylformamide), des solvants intermédiaires (par ex. acétate d'éthyle, tétrahydrofuranne) ou des solvants non polaires (par ex. toluène, hexane) et des mélanges de ceux-ci, fluides presque critiques et super-critiques (par ex. dioxyde de carbone) et/ou acides et bases tels que des acides aqueux (par ex. HCl (aq.)), des bases aqueuses (NaOH), des acides organiques (par ex. acide acétique) et des bases organiques (par ex. pyridine).

22. Procédé pour la préparation de cristaux revêtus de nanoparticules suivant l'une quelconque des revendications précédentes, dans lequel les nanoparticules revêtues sur la surface des cristaux sont accessibles pour une modification par des réactions chimiques, biochimiques ou photochimiques.

23. Procédé pour la préparation de cristaux revêtus de nanoparticules suivant l'une quelconque des revendications précédentes, dans lequel les nanoparticules revêtues agissent comme des gabarits pour une organisation de couches secondaires de manière à former une bicouche ou une multicouche de nanoparticules ou de molécules absorbées à partir d'une solution ou de la phase gazeuse.

24. Procédé pour la préparation de cristaux revêtus de nanoparticules suivant l'une quelconque des revendications précédentes, dans lequel durant le procédé de revêtement, des molécules fonctionnelles sont aussi attachées au cristal.
